**Europäisches Patentamt**

**European Patent Office**

**Office européen des brevets**

(19)

(11) Veröffentlichungsnummer: **0 085 887**
**B1**

(12) # EUROPÄISCHE PATENTSCHRIFT

(45) Veröffentlichungstag der Patentschrift:
04.06.86

(51) Int. Cl.⁴: **C 07 C 159/00**

(21) Anmeldenummer: **83100624.2**

(22) Anmeldetag: **25.01.83**

(54) **Verfahren zur Herstellung von Thiosemicarbazid.**

(30) Priorität: **06.02.82 DE 3204149**

(43) Veröffentlichungstag der Anmeldung:
**17.08.83 Patentblatt 83/33**

(45) Bekanntmachung des Hinweises auf die Patenterteilung:
**04.06.86 Patentblatt 86/23**

(84) Benannte Vertragsstaaten:
**AT BE CH DE FR GB IT LI LU NL SE**

(56) Entgegenhaltungen:
**FR - A - 1 042 274
FR - A - 1 209 045
GB - A - 1 118 133**

(73) Patentinhaber: **Degussa Aktiengesellschaft,
Weissfrauenstrasse 9, D-6000 Frankfurt am Main 1 (DE)**

(72) Erfinder: **Kleemann, Axel, Dr., Dipl.-Chem.,
Greifenhagenstrasse 25, D-6450 Hanau 9 (DE)**
Erfinder: **Klenk, Herbert, Dr. Dipl.-Chem.,
Grünaustrasse 19, D-6450 Hanau 9 (DE)**
Erfinder: **Schulz, Wolfgang, Dr., Dipl.-Chem.,
Grünaustrasse 17, DS-6450 Hanau 9 (DE)**

**Beschreibung**

Die Erfindung betrifft ein Verfahren zur Herstellung von Thiosemicarbazid durch Bildung von Hydrazinthiocyanat und dessen Überführung in das Thiosemicarbazid.

Es ist bekannt, dass sich Hydrazinthiocyanat bildet, wenn ein Salz der Thiocyansäure mit Hydrazin oder einem Salz des Hydrazins in wasserhaltigem Medium zusammengebracht wird, und dass das Hydrazinthiocyanat bei höherer Temperatur in das Thiosemicarbazid übergeht. Beispielsweise wird Ammoniumthiocyanat mit Hydrazin, Wasser und einem Alkanol mit 3 bis 5 Kohlenstoffatomen vermischt und diese Mischung nach Zusatz katalytischer Mengen Aceton zum Sieden erhitzt (GB-PS 1 118 133) oder es wird Ammoniumthiocyanat und Hydrazinsulfat in Wasser gelöst und diese Lösung nach Zugabe katalytischer Mengen Acetaldehyd zum Sieden erhitzt (DE-PS 1 274 574). Nachteilig ist bei den bekannten Verfahren, dass äquivalente Mengen Nebenprodukte, beispielsweise Ammoniak oder Ammoniumsulfat, entstehen und diese Nebenprodukte nicht oder allenfalls unter erheblichem Aufwand gewonnen und genutzt werden können.

Es ist nun ein Verfahren zur Herstellung von Thiosemicarbazid durch Bildung von Hydrazinthiocyanat und dessen Überführung in das Thiosemicarbazid gefunden worden, das dadurch gekennzeichnet ist, dass zur Bildung des Hydrazinthiocyanats Hydrazin mit Cyanwasserstoff und Schwefel umgesetzt wird. Bei diesem Verfahren entsteht das Thiosemicarbazid in ausgezeichneter Ausbeute. Besondere Vorteile des Verfahrens sind, dass unmittelbar von den einfachen Substanzen Schwefel und Cyanwasserstoff ausgegangen wird und der bei den bekannten Verfahren zwangsläufige Anfall äquivalenter Mengen von Nebenprodukten entfällt.

Zur Durchführung des erfindungsgemässen Verfahrens wird das Hydrazin mit dem Cyanwasserstoff und dem Schwefel zweckmässigerweise in Gegenwart eines organischen Lösungsmittel umgesetzt. Als Lösungsmittel kommen mit Vorteil organische Flüssigkeiten in Frage, die polar wirken, sich jedoch im übrigen inert verhalten. Derartige Lösungsmittel sind beispielsweise Alkohole oder Äther. Vorzugsweise werden Alkanole, die gegebenenfalls substituiert sind, insbesondere Alkanole mit 1 bis 4 Kohlenstoffatomen, oder cyclische Äther, insbesondere Dioxan, angewendet. Das Mengenverhältnis Hydrazin zu Lösungsmittel richtet sich in gewissem Umfang nach der Art des Lösungsmittels. Im allgemeinen ist vorteilhaft, je Gewichtsteil Hydrazin mindestens etwa zwei Gewichtsteile Lösungsmittel anzuwenden.

Die Umsetzung kann auch in Gegenwart von Wasser erfolgen, jedoch ist im allgemeinen vorteilhaft, dass je Gewichtsteil Wasser mindestens ein Gewichtsteil des organischen Lösungsmittel vorliegt. Vorzugsweise sollen je Gewichtsteil Wasser mindestens zehn Gewichtsteile des organischen Lösungsmittels vorhanden sein.

Das Hydrazin und der Cyanwasserstoff werden als solche oder als Lösungen, der Schwefel wird als solcher oder als Suspension, zweckmässigerweise in einem der genannten Lösungsmittel, eingesetzt. Vorzugsweise wird das Hydrazin als Hydrazinhydrat angewendet. Beispielsweise wird Schwefel als Suspension vorgelegt und es wird in diese Suspension Hydrazin und Cyanwasserstoff eingespeist oder es wird durch Zugabe von Cyanwasserstoff zu einer Hydrazinlösung eine Lösung von Hydrazincyanid bereitet und in diese Lösung Schwefel eingetragen.

Wenngleich das Mengenverhältnis Hydrazin zu Schwefel wie auch das Mengenverhältnis Cyanwasserstoff zu Schwefel weitgehend beliebig gewählt werden kann, ist es im allgemeinen zweckmässig, von der Stöchiometrie nicht wesentlich abzuweichen. Mit Vorteil werden etwa 1,0 bis 1,2 mol Hydrazin und etwa 1,0 bis 1,2 mol Cyanwasserstoff, besser 1,0 bis 1,1 mol Hydrazin und 1,0 bis 1,1 mol Cyanwasserstoff, je Grammatom Schwefel angewendet. Besonders geeignet sind einander äquivalente Mengen Hydrazin, Cyanwasserstoff und Schwefel.

Die Umsetzungstemperatur richtet sich gegebenenfalls nach den Mengenverhältnissen und der Art der Lösungsmittel. Im allgemeinen werden Temperaturen etwa zwischen 0 und 60 °C angewendet. Zu bevorzugen sind Temperaturen zwischen 0 und 30 °C. Der Druck kann zwar weitgehend beliebig gewählt werden, es ist jedoch vorteilhaft, vom Normaldruck nicht wesentlich abzuweichen.

Aus dem Umsetzungsgemisch wird das Hydrazinthiocyanat beispielsweise durch Abtreiben des Lösungsmittels, gegebenenfalls unter vermindertem Druck, gewonnen. Zwecks Überführung in das Thiosemicarbazid wird das Hydrazinthiocyanat in bekannter Weise bei höherer Temperatur behandelt. Vorzugsweise wird es hierzu in Wasser oder in einem organischen Lösungsmittel, insbesondere in einem der genannten polaren Lösungsmittel, oder in Gemischen eines organischen Lösungsmittels mit Wasser aufgenommen, und es werden erforderlichenfalls Aldehyde oder Ketone als Katalysatoren zugesetzt.

Im allgemeinen erfolgt die Behandlung des Hydrazinthiocyanats zwecks Überführung in das Thiosemicarbazid bei Temperaturen etwa zwischen 90 und 130 °C, vorzugsweise zwischen 95 und 110 °C. Wenngleich hierbei der Druck weitgehend beliebig gewählt werden kann, ist es vorteilhaft, vom Normaldruck nicht wesentlich abzuweichen. In manchen Fällen ist es wegen des Vorliegens flüchtiger Substanzen notwendig, einen der Temperatur entsprechenden höheren Druck zu wählen.

Eine besonders bevorzugte Verfahrensweise ist, das bei der Umsetzung des Hydrazins mit dem Cyanwasserstoff und dem Schwefel anfallende Umsetzungsgemisch unmittelbar zur Überführung des Hydrazinthiocyanats in das Thiosemicarbazid bei den höheren Temperaturen zu behandeln. In diesem Fall kann die nach der Abscheidung des Thiosemicarbazids verbleibende Mutterlauge wiederholt unmittelbar für weitere Ansätze als Lösungsmittel verwendet werden.

### Beispiel 1

Einer Lösung von 25 g (0,5 mol) Hydrazinhydrat in 30 ml Dioxan wurden zunächst tropfenweise eine Lösung von 13,5 g (0,5 mol) Cyanwasserstoff in 80 ml Dioxan und dann 16 g (0,5 Grammatome) Schwefel zugesetzt. Die Temperatur der Mischung wurde währenddessen und weitere 15 Minuten lang auf 10 °C und schliesslich 8 Stunden lang auf 90 °C gehalten. Beim Abkühlen schied sich Thiosemicarbazid ab. Der Schmelzpunkt der Substanz war 172 °C. Die Ausbeute betrug 25 g, entsprechend 55%, bezogen auf den eingesetzten Schwefel. Die nach der Abtrennung des Thiosemicarbazids verbliebene Mutterlauge wurde für einen gleichen Ansatz als Lösungsmittel verwendet. Bei diesem Ansatz betrug die Ausbeute 32 g, entsprechend 79%, bezogen auf den eingesetzten Schwefel.

### Beispiel 2a

Einer Lösung von 25 g (0,5 mol) Hydrazinhydrat in 30 ml Methanol wurden zunächst tropfenweise eine Lösung von 13,5 g (0,5 mol) Cyanwasserstoff in 80 ml Methanol und dann 16 g (0,5 Grammatome) Schwefel zugesetzt. Die Temperatur der Mischung wurde währenddessen und weitere 10 Minuten lang auf 5 °C gehalten. Dann wurde die Mischung bei 10 mbar zur Trockne eingedampft. Das hierbei als Rückstand verbliebene Hydrazinthiocyanat wurde in 120 ml Xylol aufgenommen. Die Mischung wurde bei 0,5 bar 8 Stunden auf 110 °C gehalten. Beim Abkühlen schied sich Thiosemicarbazid ab. Dieses wurde in Wasser umkristallisiert. Der Schmelzpunkt der Substanz war 174 °C. Die Ausbeute betrug 29 g, entsprechend 72%, bezogen auf den eingesetzten Schwefel.

### Patentansprüche

1. Verfahren zur Herstellung von Thiosemicarbazid durch Bildung von Hydrazinthiocyanat und dessen Überführung in das Thiosemicarbazid, dadurch gekennzeichnet, dass zur Bildung des Hydrazinthiocyanats Hydrazin mit Cyanwasserstoff und Schwefel umgesetzt wird.

2. Verfahren nach Anspruch 1, dadurch gekennzeichnet, dass die Umsetzung in einem polaren Lösungsmittel erfolgt.

### Claims

1. A process for the production of thiosemicarbazide by formation of hydrazinethiocyanate and converting it into the thiosemicarbazide, characterised in that the hydrazinethiocyanate is formed by reacting hydrazine with hydrogen cyanide and sulphur.

2. A process according to claim 1, characterised in that the reaction takes place in a polar solvent.

### Revendications

1. Procédé pour la fabrication de thiosemicarbazide, par formation du thiocyanate d'hydrazine et sa transformation en thiosemicarbazide, caractérisé en ce que, pour la formation du thiocyanate d'hydrazine, on fait réagir l'hydrazine avec de l'acide cyanhydrique et du soufre.

2. Procédé suivant la revendication 1, caractérisé en ce que la réaction s'opère dans un solvant polaire.